# EUROPEAN PATENT SPECIFICATION

(11) **EP 0 682 539 B1**
(45) Date of publication and mention of the grant of the patent: **21.07.1999**
(21) Application number: 93909561.8
(22) Date of filing: 04.05.1993
(51) Int. Cl.: A61M 15/00

(54) **DOSAGE INHALATOR WITH INDICATING/INTERRUPTING MEANS**
DOSIERINHALATOR MIT ANZEIGE-/UNTERBRECHUNGSEINRICHTUNG
INHALATION DOSEUR A ELEMENTS INDICATEUR ET D'ARRET

(30) Priority: 05.05.1992 SE 9201411
(43) Date of publication of application: 22.11.1995
(73) Proprietor: Astra Aktiebolag, 151 85 Södertälje (SE)
(72) Inventor: TROFAST, Eva, Ann-Christin, S-226 47 Lund (SE); WETTERLIN, Kjell, Ingvar, Leopold, S-240 17 Södra Sandby (SE); VIRTANEN, Risto, SF-01900 Nurmijärvi (FI)
(74) Representative: Linderoth, Margareta
(86) International application number: PCT/SE93/00389
(87) International publication number: WO 93/21980

(56) References cited:
- WO-A-91/12040
- WO-A-92/00771
- GB-A- 2 041 763
- US-A- 4 524 769
- US-A- 4 668 218

## Description

The present invention relates to a dosage inhalator for dispensing to a patient a pharmacologically active compound or substance suspended in a fluid.

Dosage inhalators of this general kind are disclosed for instance in US-A-4524769, which is directed towards a dosing means in a dosage inhalator, and US-A-4668218, which is directed towards an indicating means for indicating the number of doses used or remaining in a dosage inhalator. In some cases, it may, however, be advantageous for a patient additionally to be given a clear and unambiguous indication that the supply of active compound or substance is exhausted. It further is desirable to provide a dosage inhalator with an indicating means having a design which is simple and cheap to manufacture and which can be set automatically to correspond to the actual number of doses filled into the dosage inhalator.

Accordingly, the present invention provides a dosage inhalator for dispensing to a patient a pharmacologically active compound or substance suspended in a fluid, comprising a nozzle, a conduit connected to said nozzle, storage means adjacent said conduit for storing a plurality of doses of said active compound or substance to be dispensed, carrying means having portions for carrying predetermined and reproducible unit doses of said active compound or substance from said storage means to said conduit, said carrying means being actuated by means of a ratchet mechanism including a spring-biased pawl and mounted for intermittent movement to deliver one of said portions to said conduit, whereby said unit dose of said active compound or substance located at said portion can be dispensed into said conduit, and manoeuvering means for actuating said intermittent movement of said carrying means; characterized in that said dosage inhalator further comprises disrupting means for disrupting, in the sense to cause to break down, the normal movement of said manoeuvering means upon exhaustion of said active compound or substance from said storage means, said manoeuvering means includes a lever which in the normal movement is reciprocally displaceable between two distinct positions, and operation of said ratchet mechanism is limited by said displacement of said lever.

In the context of the present invention the term "disrupt" is used in the sense to cause to break down, for instance by locking said manoeuvering means or allowing said manoeuvering means to rotate freely in one or both rotational directions. In this way a patient is given a clear indication that the supply of active compound or substance is exhausted.

In one embodiment said disrupting means for disrupting the normal movement of said manoeuvering means places said lever in a locked state upon exhaustion of said active compound or substance from said storage means.

In another embodiment said disrupting means for disrupting the normal movement of said manoeuvering means causes said lever to be able to move freely beyond said two distinct positions upon exhaustion of said active compound or substance from said storage means.

Preferably, said dosage inhalator further comprises indicating means for indicating the number of doses remaining in said storage means or the number of doses used, said indicating means comprising a screw and nut mechanism including a screw and a nut that are moved relative to one another on intermittent movement of said carrying means, the relative movement of said screw and said nut being used to actuate said disrupting means for disrupting the normal movement of said manoeuvering means after a predetermined number of said intermittent movements.

More preferably; said screw and nut mechanism is provided with a quick adjustment feature allowing a relative translational movement between said screw and said nut which produces a corresponding movement of said indicating means to a position indicating a predetermined number of doses present in said storage means.

Still more preferably, said relative translational movement is achieved automatically by the means used for filling said dosage inhalator in response to the number of doses actually being filled.

Yet more preferably, said translational movement is achieved by means of pins of different length acting directly or indirectly upon said screw and nut mechanism by pushing one of said screw or said nut thereof to a position corresponding to the length of the pin in question.

Preferably, the threads on said screw and said nut can be disengaged from each other in order to allow said translational movement.

More preferably, said nut is a resilient biased projection which engages the threads on said screw, one of the flanks of said projection being more obliquely oriented relative to the longitudinal axis of said screw than the other flank in order to allow said projection to slide easily over the threads on said screw in one longitudinal direction but not in the opposite longitudinal direction in the manner of a ratchet mechanism.

Still more preferably, the flanks of the threads on said screw which engage said obliquely oriented flank on said projection slope conically in order to facilitate said sliding movement of said projection in said one longitudinal direction.

Preferably, said projection is located on a resilient arm.

Preferably, said nut includes a plurality of projections.

Preferred embodiments of the present invention will now be described hereinbelow by way of example only with reference to the accompanying drawings, in which:
Figure 1 illustrates a longitudinal section through a dosage inhalator in accordance with a first embodiment of the present invention,
Figure 2 illustrates a part-sectional longitudinal view of the dosage inhalator of Figure 1,
Figure 3 illustrates a diametric section through a lower pan of the dosage inhalator of Figure 1, and
Figure 4 illustrates a longitudinal section through a dosage inhalator in accordance with a second embodiment of the present invention.

The present invention will now be described hereinbelow in relation to a breath-actuated dosage inhalator of the general kind disclosed in US-A-4524769 for delivering a solid pharmacologically active compound or substance in micronized form. It will be understood that in this specification the expressions "upper", "lower", etc are used only with reference to the orientation of the dosage inhalators as illustrated and are not to be construed as limiting.

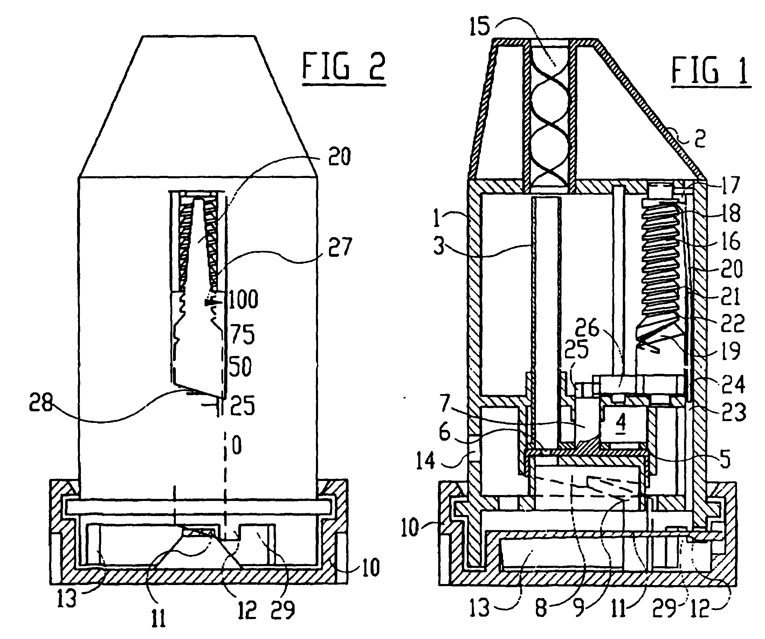

As illustrated in Figures 1 to 3, a dosage inhalator in accordance with a first embodiment of the present invention comprises a housing 1, a nozzle 2 attached to one end of the housing 1 and a rotatable manoeuvering wheel 10 journalled to the other end of the housing 1.

The housing 1 includes a conduit 3 for conducting ambient air therethrough to the nozzle 2 as a patient inhales, a storage chamber 4 for storing active compound or substance and a rotatable disc 5 for transferring the active compound or substance to the conduit 3 from the storage chamber 4. The disc 5 may be in the form of a membrane and includes one or more tapered holes 6. The disc 5 is mounted to a shaft 7 which is journalled in the housing 1 and is provided with cogs 25 at the upper end thereof. The under side of the disc 5 is provided with a ratchet wheel 8 which cooperates with a pawl 9 attached to the manoeuvering wheel 10. Movement of the manoeuvering wheel 10 relative to the housing 1 is limited by means of a lever 11 which is attached to the manoeuvering wheel 10, and first and second projections 12, 13 which act as stops that extend downwards from the wall of the housing 1. When the manouvering wheel 10 is rotated in one sense so as to move the lever 11 from the first projection 12 to the second projection 13, the ratchet wheel 8, and hence the disc 5, are rotated through an angular distance which corresponds to the angular distance between the projections 12, 13. When the manoeuvering wheel 10 is rotated back in the opposite sense so as to move the lever 11 from the second projection 13 to the first projection 12 the ratchet wheel 8, and hence the disc 5, remain stationary. The holes 6 are arranged in the disc 5 such that one or more holes 6 are located (and consequently filled) at the storage chamber 4 at the same time as one or more filled holes 6 are located at the conduit 3. Accordingly, movement of the manoeuvering wheel 10 from one stop to the other and back ensures that one or more filled holes 6 are located at the conduit 3. When a patient inhales through the nozzle 2, air will be sucked through an air intake 14, and through the one or more filled holes 6 in the conduit 3 to the nozzle 2, thus entraining the active compound or substance in the one or more holes 6 into the respiratory tract of the patient. After the dosage inhalator has been used, movement of the manoeuvering wheel 10 from one stop to the other and back, provides, as mentioned above, a new dose at the conduit 3.

In this embodiment the nozzle 2 includes a deaggregating means 15 for deaggregating any agglomerates, particularly those which form when the active compound or substance is filled into the one or more holes 6.

The dosage inhalator further comprises an indicating means for indicating the number of doses remaining in the storage chamber 4 comprising a screw 16 and a nut 17. The screw 16 comprises an upper threaded part 18 and a lower threaded part 19. The upper threaded part 18 of the screw 16 is generally clylindrical, whereas the lower threaded part 19 of the screw 16 is conical. In addition, the pitch of the threads on the upper threaded part 18 of the screw 16 is much finer than the pitch of the threads on the loner threaded part 19 of the screw 16. The upper flanks 21 of the threads on the screw 16 form an oblique angle with the longitudinal axis of the screw 16, whereas the lower flanks 22 of the threads on the screw 16 are more or less perpendicularly oriented relative to the longitudinal axis of the screw 16. In this embodiment the nut 17 is a projection that extends from a resilient arm 20 that is slideably supported in a guide 23 at the inner wall of the housing 1. Before use of the dosage inhalator, a major part of the arm 20 is located outside of the guide 23 and an upper part of the arm 20 is bent inwards tending to bias the nut 17 towards the threads on the screw 16. With use of the dosage inhalator, as the arm 20 moves downwards, an increasing length of the arm 20 is located in the guide 23 and consequently bent outwards and fixed against the wall of the housing 1, entailing that the arm 20 becomes increasingly less resilient, with the consequence that the nut 17 is held more and more fixedly relative to the screw 16. The under side of the nut 17 is oriented to be generally parallel to the upper flanks of the threads on the screw 16 and the upper side of the nut 17 is oriented to be generally parallel to the lower flanks of the threads on the screw 16. Thus, the nut 17 can be slid over the screw 16 in the manner of a ratchet mechanism to any desired position along the upper threaded part 18 of the screw 16. With use of the dosage inhalator, the nut 17 moves downwards on rotation of the screw 16 from any such position.

The screw 16 is provided with cogs 24 at the lower end thereof which cogs 24 engage with the cogs 25 on the shaft 7 by means of an intermediate cog wheel 26. Rotation of the disc 5 consequently results in rotation of the screw 16 thereby causing a downward movement of the arm 20. In this embodiment the outer side of the arm 20 is provided with a pointer 27 which is visible through a window in the housing 1 and the housing 1 is provided with a scale. With use of the dosage inhalator the arm 20 and hence the pointer 27 is moved relative to the scale and the number of doses remaining is indicated.

A visual indication that the supply of active compound or substance is close to exhaustion can be obtained in that the window has a length which is sufficient to allow the screw 16 to be seen above the arm 20 when the arm 20 is near a lower end position. In this embodiment the screw 16 is made of brightly coloured material to indicate near exhaustion of available doses.

In use, the arm 20 moves downwards at a constant rate on rotation of the disc 5 when the nut 17 is located on the upper threaded pan 18 of the screw 16. However when the nut 17 reaches the lower threaded part 19 of the screw 16, at which point the storage chamber 4 is nearly exhausted of active compound or substance, the arm 20 moves increasingly rapidly downwardly owing to the much coarser pitch of the threads on that part of the screw 16.

In the manufacture of the dosage inhalator, the nut 17 is located above the threaded parts 18, 19 of the screw 16. In this way the function of the disc 5 and the associated components can be tested before filling the storage chamber 4. After the storage chamber 4 has been filled, the nut 17 is pushed into engagement with the upper threaded part 18 of the screw 16. If the storage chamber 4 is completely filled with active compound or substance, the nut 17 is merely pushed downwards into engagement with the uppermost thread of the upper threaded part 18 of the screw 16 and the maximum number of available doses will be indicated. If, however, the storage chamber 4 is filled with a lesser amount of active compound or substance (i.e., a fewer number of doses), the nut 17 is slid further downwards over the upper threaded part 18 of the screw 16, a lesser number of available doses then being indicated. This movement of the nut 17 can be performed automatically by inserting pins of different length through a hole in the upper wall of the housing 1 directly above the guide 23. In a preferred embodiment the nut 17 is moved at the same time as the storage chamber 4 is filled.

The dosage inhalator further comprises means for disrupting the movement of the manoeuvering wheel 10 upon exhaustion of active compound or substance from the storage chamber 4, In this embodiment disruption of the manoeuvering wheel 10 is provided by the lever 11 which is acted upon by the arm 20 to locate the lever 11 immovably in a cut-out portion 29. The cut-out portion 29, which has a width corresponding to the width of the lever 11, is located in the lower edge of the wall of the housing 1 adjacent but rotationally beyond the first projection 12. As mentioned hereinabove, when the storage chamber 4 is nearly exhausted of active compound or substance, the nut 17 is located on the lower threaded part 19 of the screw 16 and the arm 20 moves rapidly downwardly on rotation of the screw 16. At this point the lower end 28 of the arm 20 moves into the path of the lever 11; the guide 23 being located rotationally immediately before the first projection 12. At the lowest position (corresponding to near exhaustion of the storage chamber 4), the lower end 28 of the arm 20, which is oriented obliquely relative to the longitudinal axis of the dosage inhalator, functions as a slideway for the resilient lever 11. The slideway provided by the lower end 28 of the arm 20 causes the lever 11 to be lifted over the first projection 12 and the lever 11 snaps down into the cut-out portion 29 beyond the first projection 12. It will be understood that the lever 11 can be locked either on loading or dispensing depending upon whether the cut-out portion 29 is located adjacent the first projection 12, as in this embodiment, or the second projection 13. In this way the maneuvering wheel 10 becomes locked upon exhaustion of active compound or substance, giving a clear indication that the dosage inhalator cannot be used further.

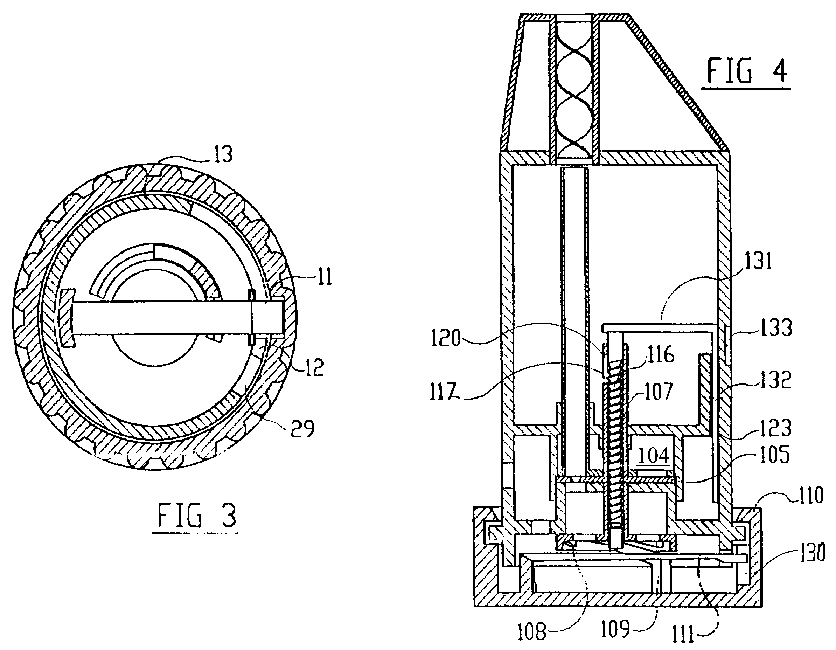

A dosage inhalator in accordance with a second embodiment of the present invention is illustrated in Figure 4. In order to avoid unnecessary duplication of description, the reference signs of the parts of the dosage inhalator of this embodiment which are the same or similar to those in the above-described embodiment will be numbered correspondingly but from 100 upwards and only the differences in the two embodiments will be described.

In this embodiment the shalt 107 to which the rotatable disc 105 is mounted is formed as a hollow sleeve which receives the screw 116. The threads on the screw 116 have the same general shape as the threads in the above-described embodiment, although in this embodiment the threads have the same pitch over the entire length of the screw 116. It will also be noted that the screw 116 is inverted relative to the screw 16 of the above-described embodiment, with the sloping flanks of the threads of the screw 116 facing downwards.

In this embodiment the arm 120 is a resilient tongue formed longitudinally in the wall of the shaft 107, with the free end of the tongue facing downwards and including a projection which faces radially inwardly and defines the nut 117. The nut 117 has the same general shape as the nut 17 in the above-described embodiment but is also inverted relative thereto. The pitch of the threads on the screw 116 is chosen such that the screw 116 will move downwards when the disc 105 is rotated by means of the ratchet mechanism provided by the ratchet wheel 108 and the pawl 109. In addition, in a similar manner to the above-described embodiment, the screw 116 can be pushed down into the shaft 107 to any desired position corresponding to a predetermined number of doses. Once set, the screw 116 will then move downwards from that position as the disc 105 is rotated.

In this embodiment the indicating means comprises a bar 132 which includes a scale that indicates the number of doses remaining and is slideable in a longitudinal guide 123 arranged on the inside of the wall of the housing 101. As in the above-described embodiment the housing 101 includes a window through which the scale is visible. The bar 132 is rigidly connected to the screw 116 by an interconnecting element 131 and is moved downwardly on rotation of the disc 105.

As described hereinabove, the screw 116 moves downwards with each rotational movement of the disc 105. When the storage chamber 104 is nearly exhausted of active compound or substance, the lower end of the screw 116 engages the resilient lever 111 and causes the lever 111 to be bent downwards. Since the screw 116 engages the lever 111 near to the attachment point thereof, a relatively small movement of the screw 116 moves the free end of the lever 111 a comparatively large distance. In the end position of the screw 116, corresponding to near exhaustion of active compound or substance from the storage chamber 104, the free end of the lever 111 is pushed down into an inner circumferential groove 130 formed in a wall portion of the maneuvering wheel 110, thereby allowing the maneuvering wheel 110 to be rotated freely without actuating the disc 105. In this way, as in the above-described embodiment a clear indication is given that the dosage inhalator is empty and cannot be used further.

Thus, a dosage inhalator is provided having an indicating means which on one hand can easily be set to show different numbers of doses of active compound or substance and which on the other hand gives a clear and unambigous indication that the active compound or substance in the dosage inhalator is exhausted and that the dosage inhalator consequently cannot be used further. Furhermore, this dosage inhalator is relatively simple and cheap to manufacture.

Finally, it will be understood that the present invention is not limited to the described embodiments, but can be modified in many different wads within the scope of the appended claims.

For instance, although the manoeuvering wheel 10, 110 and the disc 5, 105 in the above-described embodiments are separate parts, it is envisaged that the manoeuvering wheel 10, 110 and the disc 5, 105 could be formed as a single component. For example, the manoeuvering wheel 10, 110 could be provided as a knurled portion of the disc 5, 105 which extends through a circumferential slot in the housing 1, 101.

Furthermore, it is envisaged that the scale could be included on either the housing 1, 101 or one of the arm 20 and the bar 132, with the pointer on the other of the housing 1, 101 or one of the arm 20 and the bar 132.

Another possible modification of the above-described embodiments is to arrange the threads on the screw 16, 116 and the nut 17, 117 to allow those parts to be disengaged from each other in order to permit translational movement.

## Claims

1. A dosage inhalator for dispensing to a patient a pharmacologically active compound or substance suspended in a fluid, comprising a nozzle (2), a conduit (3) connected to said nozzle (2), storage means (4) adjacent said conduit (3) for storing a plurality of doses of said active compound or substance to be dispensed, carrying means (5; 105) having portions (6) for carrying predetermined and reproducible unit doses of said active compound or substance from said storage means (4) to said conduit (3), said carrying means (5; 105) being actuated by means of a ratchet mechanism including a spring-biased pawl (9; 109) and mounted for intermittent movement to deliver one of said portions (6) to said conduit (3), whereby said unit dose of said active compound or substance located at said portion (6) can be dispensed into said conduit (3), and manoeuvering means (110; 110) for actuating said intermittent movement of said carrying means (5; 105); characterized in that said dosage inhalator further comprises disrupting means for disrupting, in the sense to cause to break down, the normal movement of said manoeuvering means (10; 110) upon exhaustion of said active compound or substance from said storage means (4), said manoeuvering means (10; 110) includes a lever (11; 111) which in the normal movement is reciprocally displaceable between two distinct positions, and operation of said ratchet mechanism is limited by said displacement of said lever (11; 111).

2. A dosage inhalator according to claim 1, wherein said disrupting means for disrupting the normal movement of said manoeuvering means (10) places said lever (11) in a locked state upon exhaustion of said active compound or substance from said storage means (4),

3. A dosage inhalator according to claim 1, wherein said disrupting means for disrupting the normal movement of said manoeuvering means (110) causes said lever (111) to be able to move freely beyond said two distinct positions upon exhaustion of said active compound or substance from said storage means (4).

4. A dosage inhalator according to any of claims 1 to 3, further comprising indicating means for indicating the number of doses remaining in said storage means (4) or the number of doses used, said indicating means comprising a screw and nut mechanism including a screw (16; 116) and a nut (17; 117) that are moved relative to one another on intermittent movement of said carrying means (5; 105), the relative movement of said screw (16; 116) and said nut (17; 117) being used to actuate said disrupting means for disrupting the normal movement of said manoeuvering means (10; 110) after a predetermined number of said intermittent movements.

5. A dosage inhalator according to claim 4, wherein said screw and nut mechanism is provided with a quick adjustment feature allowing a relative translational movement between said screw (16; 116) and said nut (17; 117) which produces a corresponding movement of said indicating means to a position indicating a predetermined number of doses present in said storage means (4).

6. A dosage inhalator according to claim 5, wherein said relative translational movement is achieved automatically by the means used for filling said dosage inhalator in response to the number of doses actually being filled.

7. A dosage inhalator according to claim 6, wherein said translational movement is achieved by means of pins of different length acting directly or indirectly upon said screw and nut mechanism by pushing one of said screw (16; 116) or said nut (17; 117) thereof to a position corresponding to the length of the pin in question.

8. A dosage inhalator according to any of claims 5 to 7, wherein the threads on said screw (16; 116) and said nut (17; 117) can be disengaged from each other in order to allow said translational movement.

9. A dosage inhalator according to claim 8, wherein said nut (17; 117) is a resilient biased projection which engages the threads on said screw' (16; 116), one of the flanks of said projection being more obliquely oriented relative to the longitudinal axis of said screw (16; 116) than the other flank in order to allow said projection to slide easily over the threads on said screw (16; 116) in one longitudinal direction but not in the opposite longitudinal direction in the manner of a ratchet mechanism.

10. A dosage inhalator according to claim 9, wherein the flanks of the threads on said screw (16; 116) which engage said obliquely oriented flank on said projection slope conically in order to facilitate said sliding movement of said projection in said one longitudinal direction.

11. A dosage inhalator according to claim 9 or 10 wherein said projection is located on a resilient arm (20; 120).

12. A dosage inhalator according to any of claims 9 to 11 wherein said nut (17; 117) includes a plurality of projections.

## Patentansprüche

1. Dosierinhalator zur Abgabe eines in einer Flüssigkeit suspendierten pharmakologischen Wirkstoffes oder einer solchen pharmakologischen Wirksubstanz an einem Patienten, mit einer Düse (2), einer mit der Düse (2) verbundenen Leitung (3), einer Speicheranordnung (4) neben der Leitung (3) zum Speichern mehrerer Dosen des abzugebenden Wirkstoffes oder der abzugebenden Wirksubstanz, einer Beförderungsanordnung (5; 105) mit Teilen (6) zur Beförderung vorbestimmter und reproduzierbarer Dosiereinheiten des Wirkstoffes oder der Wirksubstanz von der Speicheranordnung (4) zu der Leitung (3), wobei die Beförderungsanordnung (5; 105) durch einen Sperrmechanismus betätigt wird, der eine federbelastete Sperrklinke (9; 109) enthält und zur Ausführung einer intermittierenden Bewegung, um der Leitung (3) einen der Teile (6) zuzuführen, wodurch die sich an dem Teil (6) befindende Dosiereinheit des Wirkstoffes oder der Wirksubstanz in die Leitung (3) abgegeben werden kann, angebracht ist, und einer Betätigungsanordnung (10; 110) zur Bewirkung der intermittierenden Bewegung der Beförderungsanordnung (5; 105); dadurch gekennzeichnet, daß der Dosierinhalator weiterhin eine Unterbrechungsanordnung zur Bewirkung einer Unterbrechung, im Sinne einer Störung, der normalen Bewegung der Betätigungsanordnung (10; 110) bei Verbrauch des Wirkstoffes oder der Wirksubstanz aus der Speicheranordnung (4) umfaßt, die Betätigungsanordnung (10; 110) einen Hebel (11; 111) enthält, der bei der normalen Bewegung zwischen zwei verschiedenen Positionen hin- und herbewegt werden kann, und der Betrieb des Sperrmechanismus durch die Bewegung des Hebels (11; 111) begrenzt wird.

2. Dosierinhalator nach Anspruch 1, bei dem die Unterbrechungsanordnung zur Unterbrechung der normalen Bewegung der Betätigungsanordnung (10) den Hebel (11) bei Verbrauch des Wirkstoffes oder der Wirksubstanz aus der Speicheranordnung (4) in einen verriegelten Zustand anordnet.

3. Dosierinhalator nach Anspruch 1, bei dem die Unterbrechungsanordnung zur Unterbrechung der normalen Bewegung der Betätigungsanordnung (110) bewirkt, daß sich der Hebel (111) bei Verbrauch des Wirkstoffes oder der Wirksubstanz aus der Speicheranordnung (4) frei über die beiden verschiedenen Positionen hinaus bewegen kann.

4. Dosierinhalator nach einem der Ansprüche 1 bis 3, weiterhin mit einer Anzeigeanordnung zur Anzeige der Anzahl von in der Speicheranordnung (4) verbleibenden Dosen oder der Anzahl verwendeter Dosen, wobei die Anzeigeanordnung einen Schrauben- und Mutternmechanismus umfaßt, der eine Schraube (16; 116) und eine Mutter (17; 117) enthält, die bei intermittierender Bewegung der Beförderungsanordnung (5; 105) relativ zueinander bewegt werden, wobei die Relativbewegung der Schraube (16; 116) und der Mutter (17; 117) zur Betätigung der Unterbrechungsanordnung zur Unterbrechung der normalen Bewegung der Betätigungsanordnung (10; 110) nach einer vorbestimmten Anzahl der intermittierenden Bewegungen verwendet wird.

5. Dosierinhalator nach Anspruch 4, bei dem der Schrauben- und Mutternmechanismus mit einem Schnelleinstellmerkmal versehen ist, das eine translatorische Relativbewegung zwischen der Schraube (16; 116) und der Mutter (17; 117) gestattet, welche eine entsprechende Bewegung der Anzeigeanordnung in eine Position erzeugt, die eine vorbestimmte Anzahl von in der Speicheranordnung (4) vorhandenen Dosen anzeigt.

6. Dosierinhalator nach Anspruch 5, bei dem die translatorische Relativbewegung durch die zum Füllen des Dosierinhalators als Reaktion auf die Anzahl von tatsächlich eingefüllten Dosen verwendete Anordnung automatisch erreicht wird.

7. Dosierinhalator nach Anspruch 6, bei dem die translatorische Bewegung mittels Stiften unterschiedlicher Länge, die direkt oder indirekt auf den Schrauben- und Mutternmechanismus einwirken, indem sie entweder die Schraube (16; 116) oder die Mutter (17; 117) davon in eine Position schieben, die der Länge des betreffenden Stifts entspricht, erreicht wird.

8. Dosierinhalator nach einem der Ansprüche 5 bis 7, bei dem die Gewinde an der Schraube (16; 116) und an der Mutter (17; 117) außer Eingriff voneinander gebracht werden können, um die translatorische Bewegung zu gestatten.

9. Dosierinhalator nach Anspruch 8, bei dem die Mutter (17; 117) ein elastisch vorbelasteter Vorsprung ist, der das Gewinde an der Schraube (16; 116) in Eingriff nimmt, wobei eine der Flanken des Vorsprungs bezüglich der Längsachse der Schraube (16; 116) schräger ausgerichtet ist als die andere Flanke, damit der Vorsprung auf die Weise eines Sperrmechanismus leicht über das Gewinde an der Schraube (16; 116) in eine Längsrichtung, aber nicht in die entgegengesetzte Längsrichtung gleiten kann.

10. Dosierinhalator nach Anspruch 9, bei dem die Flanken des Gewindes an der Schraube (16; 116), die die schräg ausgerichtete Flanke am Vorsprung in Eingriff nehmen, konisch abfallen, um die Gleitbewegung des Vorsprungs in die eine Längsrichtung zu erleichtern.

11. Dosierinhalator nach Anspruch 9 oder 10, bei dem sich der Vorsprung an einem elastischen Arm (20; 120) befindet.

12. Dosierinhalator nach einem der Ansprüche 9 bis 11, bei dem die Mutter (17; 117) mehrere Vorsprünge enthält.

## Revendications

1. Inhalateur doseur pour administrer à un patient un composé actif ou substance active sur le plan pharmacologique en suspension dans un fluide, comprenant une embouchure (2), un conduit (3) raccordé à ladite embouchure (2), un moyen de stockage (4) attenant audit conduit (3) pour stocker une pluralité de doses de dudit composé actif ou de ladite substance active à administrer, un moyen d'acheminement (5; 105) comportant des parties (6) pour acheminer des doses unitaires prédéterminées et reproductibles dudit composé actif ou de ladite substance active à partir dudit moyen de stockage (4) vers ledit conduit (3), ledit moyen d'acheminement (5; 105) étant actionné au moyen d'un mécanisme d'encliquetage comprenant un cliquet à ressort (9; 109) et étant monté en vue d'un déplacement intermittent pour amener l'une desdites parties (6) audit conduit (3), de sorte que ladite dose unitaire dudit composé actif ou de ladite substance active placé(e) au niveau de ladite partie (6) puisse être débitée dans ledit conduit (3), et un moyen de manoeuvre (10; 110) pour actionner ledit déplacement intermittent dudit moyen d'acheminement (5; 105), caractérisé en ce que ledit inhalateur doseur comprend en outre un moyen d'interruption pour interrompre, dans le sens de l'arrêter, le mouvement normal dudit moyen de manoeuvre (10; 110) lors de l'épuisement dudit composé actif ou de ladite substance active provenant dudit moyen de stockage (4), en ce que ledit moyen de manoeuvre (10; 110) comprend un levier (11; 111) qui, lors du mouvement normal, peut être déplacé en va-et-vient entre deux positions distinctes, et en ce que le fonctionnement dudit mécanisme d'encliquetage est limité par ledit déplacement dudit levier (11; 111).

2. Inhalateur doseur suivant la revendication 1, dans lequel ledit moyen d'interruption pour interrompre le mouvement normal dudit moyen de manoeuvre (10) place ledit levier (11) dans un état bloqué lors de l'épuisement dudit composé actif ou de ladite substance active provenant dudit moyen de stockage (4).

3. Inhalateur doseur suivant la revendication 1, dans lequel ledit moyen d'interruption pour interrompre le mouvement normal dudit moyen de manoeuvre (110) amène ledit levier (111) à être à même de se déplacer librement au-delà desdites deux positions distinctes lors de l'épuisement dudit composé actif ou de ladite substance active provenant dudit moyen de stockage (4).

4. Inhalateur doseur suivant l'une quelconque des revendications 1 à 3, comprenant en outre un moyen d'indication pour indiquer le nombre de doses restant dans ledit moyen de stockage (4) ou le nombre de doses utilisées, ledit moyen d'indication comprenant un mécanisme à vis et à écrou comprenant une vis (16; 116) et un écrou (17; 117) qui sont déplacés l'un par rapport à l'autre lors du déplacement intermittent dudit moyen d'acheminement (5; 105), le déplacement relatif de ladite vis (16; 116) et dudit écrou (17; 117) étant utilisé pour actionner ledit moyen d'interruption pour interrompre le déplacement normal dudit moyen de manoeuvre (10; 110) après un nombre prédéterminé desdits déplacements intermittents.

5. Inhalateur doseur suivant la revendication 4, dans lequel ledit mécanisme à vis et à écrou présente une possibilité de réglage rapide permettant un mouvement de translation relatif entre ladite vis (16; 116) et ledit écrou (17; 117) qui produit un mouvement correspondant dudit moyen d'indication vers une position indiquant un nombre prédéterminé de doses présentes dans ledit moyen de stockage (4).

6. Inhalateur doseur suivant la revendication 5, dans lequel ledit mouvement de translation relatif est obtenu automatiquement par le moyen utilisé pour remplir ledit inhalateur doseur en réaction au nombre de doses réellement en cours de remplissage.

7. Inhalateur doseur suivant la revendication 6, dans lequel ledit mouvement de translation est obtenu au moyen d'ergots de longueurs différentes agissant directement ou indirectement sur ledit mécanisme à vis et à écrou en poussant ladite vis (16; 116) ou ledit écrou (17; 117) vers une position correspondant à la longueur de l'ergot en question.

8. Inhalateur doseur suivant l'une quelconque des revendications 5 à 7, dans lequel les filets sur ladite vis (16; 116) et ledit écrou (17; 117) peuvent être mis réciproquement hors de prise afin de permettre ledit mouvement de translation.

9. Inhalateur doseur suivant la revendication 8, dans lequel ledit écrou (17; 117) est une saillie sollicitée élastiquement qui vient en prise avec les filets sur ladite vis (16; 116), un des flancs de ladite saillie étant orienté davantage en oblique par rapport à l'axe longitudinal de ladite vis (16; 116) que l'autre flanc, afin de permettre à ladite saillie de coulisser aisément sur les filets de ladite vis (16; 116) dans une direction longitudinale, mais pas dans la direction longitudinale opposée à la manière d'un mécanisme d'encliquetage.

10. Inhalateur doseur suivant la revendication 9, dans lequel les flancs des filets sur ladite vis (16; 116) qui viennent en prise avec ledit flanc orienté en oblique sur ladite saillie sont inclinés de manière conique afin de faciliter ledit mouvement de coulissement de ladite saillie dans ladite une direction longitudinale.

11. Inhalateur doseur suivant la revendication 9 ou 10, dans lequel ladite saillie se trouve sur un bras élastique (20; 120).

12. Inhalateur doseur suivant l'une quelconque des revendications 9 à 11, dans lequel ledit écrou (17; 117) comprend une pluralité de saillies.
